# EUROPEAN PATENT SPECIFICATION

(11) **EP 0 955 954 B1**
(45) Date of publication and mention of the grant of the patent: **16.03.2005**
(21) Application number: 97901347.1
(22) Date of filing: 03.01.1997
(51) Int. Cl.: A61F 2/06

(54) **EXPANSIBLE ENDOLUMINAL PROSTHESES**
EXPANDIERBARE ENDOLUMINALE PROTHESEN
PROTHESES ENDOLUMINALES EXPANSIBLES

(30) Priority: 05.01.1996 US 583814; 06.02.1996 US 595944; 10.06.1996 US 19483 P
(43) Date of publication of application: 17.11.1999
(73) Proprietor: Medtronic, Inc., Minneapolis, Minnesota 55432-5604 (US)
(72) Inventor: LENKER, Jay, A., Los Altos Hills, CA 94024 (US); WEINBERG, Steven, League City, TX 77573 (US); COX, Brian, J., Los Altos, CA 94024 (US); EVANS, Michael, A., Palo Alto, CA 94301 (US)
(74) Representative: Henseler, Daniela, Dr.
(86) International application number: PCT/US1997/000137
(87) International publication number: WO 1997/025002

(56) References cited:
- EP-A- 0 122 744
- EP-A- 0 686 379
- WO-A-88/00813
- US-A- 3 304 557
- US-A- 3 316 557
- US-A- 5 556 413

## Description

### BACKGROUND OF THE INVENTION

### 1. Field of the Invention

The present invention relate to a controlled expansion endoluminal prothesis according to the preamble of claim 1 and thus generally to tubular prostheses, such as grafts, stents, stent-grafts, and the like. More particularly, the present invention provides radially expansible tubular prosthetic structures which can be expanded to match individual body lumens, including blood vessels, particularly for the treatment of abdominal and other aneurysms.

Vascular aneurysms are the result of abnormal dilation of a blood vessel, usually resulting from disease and/or genetic predisposition, which can weaken the arterial wall and allow it to expand. While aneurysms can occur in any blood vessel, most occur in the aorta and peripheral arteries, with the majority of aortic aneurysms occurring in the abdominal aorta, usually beginning below the renal arteries and often extending into one or both of the iliac arteries.

Aortic aneurysms are most commonly treated in open surgical procedures where the diseased vessel segment is bypassed and repaired with an artificial vascular graft. While considered to be an effective surgical technique, particularly considering the alternative of a usually fatal ruptured abdominal aortic aneurysm, conventional vascular graft surgery suffers from a number of disadvantages. The surgical procedure is complex and requires experienced surgeons and well equipped surgical facilities. Ever, with the best surgeons and equipment, however, patients being treated frequently are elderly and weakened from cardiovascular and other diseases, reducing the number of eligible patients. Even for eligible patients prior to rupture, conventional aneurysm repair has a relatively high mortality rate, usually from 2% to 10%. Morbidity related to the conventional surgery includes myocardial infarction, renal failure, impotence, paralysis, and other conditions. Additionally, even with successful surgery, recovery takes several weeks, and often requires a lengthy hospital stay.

In order to overcome some or all of these drawbacks, endovascular prosthesis placement for the treatment of aneurysms has been proposed. Although very promising, many of the proposed methods and apparatus suffer from undesirable limitations. In particular, proper sizing of endovascular prostheses can be problematic.

Proper matching of the prosthesis to the blood vessel is critical to the treatment of an aneurysm. The prosthesis preferably extends axially beyond the weakened portion of the blood vessel to anchor securely in the healthy vessel wall. However, the cross-sectional size and axial length of individual blood vessels vary considerably between patients. Even within a patient, the cross-section and resilience of a lumenal wall can vary considerably along its axial length, and the location and extent of the aneurysm will differ with different patients. Additionally, each prosthesis must be carefully constructed and handled, making it extremely costly to provide and maintain the large selection of prostheses required for proper fitting of every individual patient.

Known radially expandable endoluminal prostheses may generally be characterized as either resilient or plastically expandable structures. Resilient endoluminal prostheses are often formed as stent-grafts having self-expanding frames or "stents" which radially conform to irregular lumenal cross-sections. Such resilient stent-grafts must expand against the luminal wall with sufficient force to anchor the prosthesis within the body lumen, and should ideally be sealed around the perimeter of the luminal wall to prevent leakage. Resilient prostheses which are too small may not expand sufficiently to seal or anchor properly, while oversized resilient prostheses can exert excessive pressure against the surrounding body lumen.

Plastically expansible endoluminal prostheses have malleable frames which are expanded to fit the lumen when implanted. Unfortunately, the expanded prosthesis generally takes the cylindrical shape of the expanding balloon catheter, rather than conforming to irregular luminal cross-sections. Additionally, the expanded prosthesis must be sufficiently large and rigid to provide a stable anchor and perimeter seal, requiring distension of the lumen adjacent the disease condition. Hence, even with ideal fitting, most resilient or plastically expansible prostheses impose some stress on the body lumen. A still further complication arises from the use of a separate liner or "graft," which is often woven from inexpansible polyesters such as Dacron™, and which may therefore wrinkle and occlude the lumen if the stent-graft is not fully expanded.

It has previously been proposed to use radially expansible liners with plastically expansible stents so that the liner and the frame may be expanded together within a body lumen. In particular, liner materials having undrawn or partially drawn yarns in the circumferential direction allow plastic expansion of the liner and frame using a balloon catheter. Such liner materials would thus facilitate *in situ* expansion of plastically expandable stent-grafts within a wider range of sizes.

Unfortunately, known prostheses having plastically expansible liner materials suffer from several disadvantages. As described above, such prostheses generally also include frames which are rigid when expanded, typically relying on distension of the body lumen around a cylindrical frame to anchor and seal the prosthesis. Furthermore, undrawn or partially drawn liners may be inadvertently overexpanded, resulting in "creeping" of the material, changes in porosity, or even in the creation of open fistulas during deployment or resizing of the prosthesis. Any such overexpansion of the liner might well go undetected, as *in situ* expansion is generally a fluoroscopically directed process in which the condition of the liner is not easily monitored.

Additionally, because of the great expansibility of partially drawn yarns, any bulges formed by uneven expansion of the liner material may continue to expand in an uncontrolled manner during deployment or size adjustment. Such bulges in the liner may even result in a weak, oversized region that could potentially collect thrombus or even fail during deployment--effectively resulting in an aneurysm of the prosthesis. Such bulges in an endoluminal prosthesis may also cause folds of the liner material, leading to leakage between the prosthesis and the vessel wall.

Co-pending U.S. Patent Application Serial No. 08/538,706, which is assigned to the assignee of the present application, describes a resiliently expandable prosthesis which includes a plastically expansible liner with a resilient frame, in which the resilient expansion of the frame is restrained by the liner. Advantageously, such a liner-restrained structure allows *in situ* expansion of the liner to match the perimeter of the surrounding body lumen, and also allows the fitted prosthesis to resiliently conform to irregular lumenal cross-sections. Application Serial No. 08/538,706 also teaches the selective expansion of "sealing cuffs," integral or separate prosthetic end seals, which preferably include expansible liner materials to facilitate sealing and conforming an end of a tubular prosthesis against the surrounding body lumen wall. The use of liner materials with partially oriented yarns was suggested for these liner-restrained prostheses and sealing cuffs.

Although the expandable prosthetic structures described above provide substantial advantages over other endoluminal prostheses, still further refinements are desirable. In general, it would be desirable to provide improved prostheses, including grafts and stent-grafts, and improved methods for placement of such prostheses to treat aneurysms and other conditions. It would be particularly desirable to provide frame structures and liner materials for use in liner-restrained and other endoluminal prostheses which would allow the prosthesis to expand plastically within a preset range, but which would reduce the danger of overexpansion. It would further be advantageous to provide liner materials which would allow controlled, selective expansion of portions of the prosthesis to promote anchoring or sealing, but which would resist expansion in alternative portions, particularly adjacent a weakened portion of a body lumen.

### 2. Description of the Background Art

U.S. Patent No. 5,443,499 describes a radially expandable tubular prosthesis formed with radial yarns which are at most partially drawn. The prosthesis may optionally be secured to a body lumen through simultaneous balloon expansion of the prosthesis and an attached stent.

U.S. Patents No. 3,853,462 and 3,986,828 describe methods for compaction of polymer and polyester fabric materials. P.C.T. Patent Application WO 88/00,813 describes a braided polyester vascular prosthesis and method.

French Patent Application Publication No. FR 2,714,816 describes a vascular prosthesis including a sleeve which contracts axially when stretched radially. Sliding connections are provided between a support structure and the sleeve, and additional material is preferably provided to compensate for axial contraction of the sleeve. Similarly, U.S. Patent No. 5,064,435 describes a self expanding prosthesis which maintains a stable axial length during expansion by anchoring of radially outward flares at each end, and by sliding of an overlapping medial region therebetween. U.S. Patent No. 4,834,755 describes a triaxially-braided fabric prosthesis structure to provide controlled strength and elasticity. U.S. Patent No. 5,456,713 is generally relevant.

U.S. Patent No. 5,258,042 describes an intravascular hydrogel implant which expands when hydrated. U.S. Patent No. 5,470,313 describes a variable diameter balloon dilation catheter having a pressure controlled inflation profile.

EP-A-0 686 379, with respect to which claim 1 is delimited, describes a vascular graft comprising a tubular expansible frame and a liner.

### SUMMARY OF THE INVENTION

The invention is defined in claim 1. Embodiments are defined in the dependent claims.

The present invention generally provides radially expansible tubular prostheses, particularly grafts, stents, and stent-grafts, for the treatment of aneurysms, stenoses, and other disease conditions. The expansible prostheses of the present invention may be tailored by selective mechanical expansion of limited regions of the prosthesis, or may alternatively be uniformly radially expanded, depending upon the specific requirements of the patient. *In situ* expansion is limited by an element of the prosthesis, e.g., by inelastic circumferential fibers of a prosthetic liner, or by a circumferential element of the frame which limits expansion, so as to avoid overexpansion of a portion of the liner. Such a self-limiting expansible structure will thus minimize the possibility of overexpanding the prosthetic lumen.

The controlled expansion of the present prostheses is generally limited by a structural element of the prosthesis itself. Hence, a stent-graft according to the invention will often expand, either resiliently or plastically, only to some predetermined expansion limit, at which limit an element of either the liner or the frame (or both) impedes further expansion. In some cases, the expansion limit will be present in or provided on the interface between the liner and the frame, for example, a circumferential band of suture or other material which both limits stent-graft expansion and connects the liner to the frame.

The expansion limit itself may provide either a fixed radial limit or an intermediate limit. A fixed limit prevents any significant expansion of the prosthesis beyond a maximum safe distention cross-section for the body lumen, regardless of the expansive radial force applied to the prosthesis. Such a fixed limit may also help to prevent local or global porosity of the liner from exceeding a desired maximum, to avoid the creation of fistulas, and to promote even expansion of the prosthesis, rather than bulging of any weak regions. Intermediate expansion limits will provide some mechanism which allows expansion to continue beyond an initial limit, for example, by incorporating a frangible or plastic reinforcing element in the frame or liner which fails or plastically deforms under a threshold expansive force. Intermediate limits thereby provide the safety of an expansion limitation, but with the added option of continued expansion when justified. Optionally, a plurality of intermediate limitations may be used in series, or in combination with a fixed limit.

Additionally, in work in connection with the present invention, it has been discovered that chemical compaction of endoluminal prosthetic liner materials, particularly fully drawn polyester fibers, can greatly enhance their plastic expansibility. Chemical compaction appears to change the crystallinity of the fibers, creating amorphous zones within the oriented polymer structure, resulting in mechanical characteristics that provide controllable expansibility. Hence, endoluminal prostheses having circumferentially oriented, chemically compacted, fully drawn fibers will be plastically radially expansible. Advantageously, the fibers may be compacted before weaving, or the liner fabric may be compacted, and extensibility can be locally or globally enhanced by controlling the density of the weave.

As used herein, "expansible" generally refers to both self-expanding structures which increase in dimensions when released from compression or subjected to a change of state (e.g., shape recovery of shape memory alloys), and also to structures which deform plastically when subjected to expansive stress. Hence, both resilient and plastically expansible (sometimes called malleable) stints are encompassed by the term "expansible frames." In contrast, "plastically expansible" herein more specifically refers to structures which plastically increase in dimension when under an expansive force.

As used herein, a "fill element" means fiber, monofilament, fiber within a thread, fiber within a yarn, a thread within a yarn, or, alternatively, a yarn itself, which forms a circumferentially oriented element of the liner material.

In a first aspect, the present invention provides a controlled expansion endoluminal prothesis comprising a radially expansible tubular frame and a plastically expansible liner on the frame. Either the frame or the liner includes a reinforcing element which limits expansion of the prosthesis at a predetermined expanded size. Generally, the reinforcing element is included in the liner as circumferentially oriented yarn. A particularly advantageous reinforced liner includes composite circumferential yarns having inexpansible fibers wrapped around an expansible fiber, such as a partially oriented fiber, PTFE, or the like. In other embodiments, the reinforcing element restrains the frame, for example, by limiting the expansion of individual perforations on a perforate frame structure.

In contrast to known expansible liner materials, the liner may comprise a fill element including fully drawn fiber which defines a maximum expanded perimeter of the liner. The fully drawn fiber may optionally wind around another fiber so as to straighten during expansion, or may alternatively be texturized or annealed after it is drawn. Advantageously, such fibers will substantially retain the ultimate strength of the fully drawn fiber.

The liner may comprise a fabric tube which includes composite yarns. The composite yarns include both serpentine inexpansible fiber and expansible fiber so that the inexpansible fiber straightens when the graft is intentionally expanded. The straightening inexpansible fiber gradually becomes taut, thereby preventing expansion of the graft beyond a predetermined limit. Preferably, the inexpansible fiber is wound over the expansible fiber. Advantageously, the expansible fiber or "core" generally maintains structural integrity of the liner to prevent inadvertent deformation or distention of the prosthesis under physilogical stresses.

The endoluminal prosthesis may be deployed at a target site within a diseased body lumen by positioning the prosthesis at the target site, at which a liner of the prosthesis is plastically expanded. Advantageously, the plastic expansion of the liner is limited to a predetermined size by an element of the prosthesis.

The endoluminal prosthesis may also be deployed at a target site within a diseased body lumen by introducing the prosthesis into the body lumen and positioning the prosthesis at the target site. A phase of a reinforcement element of the prosthesis is altered at the target site to increase expansibility of the element, and the cross-section of the prosthesis is expanded while the phase remains altered. The phase of the reinforcement element is then returned to reduce expansibility. Generally, the phase altering step comprises changing a temperature of a temperature sensitive polymer, which is ideally included in a circumferentially oriented fiber and woven into a prosthesis liner.

The prosthesis may be produced by drawing fiber to a fully drawn length and then texturizing the drawn fiber. The texturized fiber may then be woven into a tube so that the fiber is circumferentially oriented.

The prosthesis may also be produced by drawing fiber to a fully drawn length and annealing the drawn fiber. The fiber is preferably woven into a tube so that the fiber is circumferentially oriented.

The radially expansible tubular frame may define an axial direction and a circumferential direction and support an expansible liner, the liner comprising a fabric tube which includes circumferentially oriented fibers that have been chemically compacted. Surprisingly, the circumferentially oriented fibers may comprise fully oriented polyester, providing radial expansibility with the recognized biocompatibility and ingrowth characteristics of this otherwise inexpansible liner material.

The tubular fabric liner may comprise a circumferentially oriented fully drawn polymer fiber. The radial expansibility of the liner is enhanced by forming amorphous zones within a crystalline structure of the fully drawn polymer fiber, and the liner is affixed to a radially expansible frame.

### BRIEF DESCRIPTION OF THE DRAWINGS

Fig. 1 is a side view of an exemplary vascular stent-graft having an expansible liner according to the principles of the present invention.
Fig. 2 is a perspective view of an exemplary delivery catheter for use with the prostheses of Fig. 1, with a portion of the distal end broken away to disclose a prosthesis therein.
Fig. 3 illustrates a modular branching intraluminal prosthesis assembly from expansible prosthetic modules according to the principles of the present invention.
Fig. 4 is a schematic illustration of a method for selectively expanding an integral prosthetic sealing cuff.
Figs. 5 and 5A illustrate frame limited stent-grafts having circumferential belts which limit radial expansion, according to the principals of the present invention.
Figs. 5B-E illustrate ring-frames having expansion limiting elements which releasably restrain expansion of the liner at a predetermined size.
Fig. 6 is a cross-section of an endovascular stent-graft having an expansible liner wherein low strength attachments releasably maintain a plurality of axial folds.
Fig. 7 is a perspective view of a radially expansible woven vascular graft having circumferentially oriented fibers which are expansible, according to the principles of the present invention.
Figs 8A and B illustrate a composite yarn having inelastic fiber wrapped around a core fiber so that the inelastic fiber straightens and limits total elongation of the yarn, for use as a circumferentially oriented yarn in the graft of Fig. 7.
Figs. 9A and B illustrate alternative wrapped composite yarn structures, for use as a circumferentially oriented yarn in the graft of Fig. 7.
Fig. 10 illustrates a method for producing annealed fully drawn fibers, for use as a circumferentially oriented yarn in the graft of Fig. 7.
Figs. 11A and 11B illustrate alternative integral sealing cuffs having a braided sealing structure.
Figs. 12-13 illustrate balloon catheters having a plurality of chambers and methods for their use to grip and selectively expand an integral sealing cuff of an endoluminal prosthesis.
Fig. 14 schematically illustrates a method for compacting the graft of Fig. 7 to promote radial expansibility.
Fig. 15 schematically illustrates a system and method for compacting fiber which is later woven to form the graft of Fig. 7.
Figs. 15A and B schematically illustrate a collapsible cone and an associated fixture for bulk compaction of fiber for use in the graft of Fig. 7.
Figs. 16A-C illustrate an alternative circumferentially oriented fiber for use in the graft of Fig. 7, and a method for its fabrication, in which an inexpansible fiber is wrapped around a core fiber to form a helical coil, and the core is then at least partially dissolved or weakened.
Figs 17-19 illustrate selectively varying the weave density of a liner to provide local control over expansibility and minimize the total porosity of the prosthesis.
Fig. 20 schematically illustrates an endoluminal prosthesis formed as a fabric tube having a temperarature sensitive polymer dispersion and a method for its production.

### DESCRIPTION OF THE SPECIFIC EMBODIMENTS

The present invention provides radially expansible tubular prostheses, particularly grafts, stents, and stent-grafts. Methods for their production, and methods for their deployment are described herein for a full understanding of all aspects of the invention. The prostheses of the present invention are suitable for a wide variety of therapeutic uses, including stenting of the ureter, urethra, biliary tract, and the like. The present devices and methods will also be useful for the creation of temporary or long term lumens, such as the formation of fistulas. The prosthetic structures of the present invention will find their most immediate use as endovascular prostheses for the treatment of diseases of the vasculature, particularly aneurysms, stenoses, and the like. These prostheses will generally be radially expansible from a narrow-diameter configuration to facilitate introduction into the body lumen, typically during surgical cutdown or percutaneous introduction procedures. The present expansible structures and materials will also find particular use as separate or integral sealing elements disposed at the ends of a prosthetic lumen, or as conduits to provide atraumatic seals between the prosthesis and the body lumen.

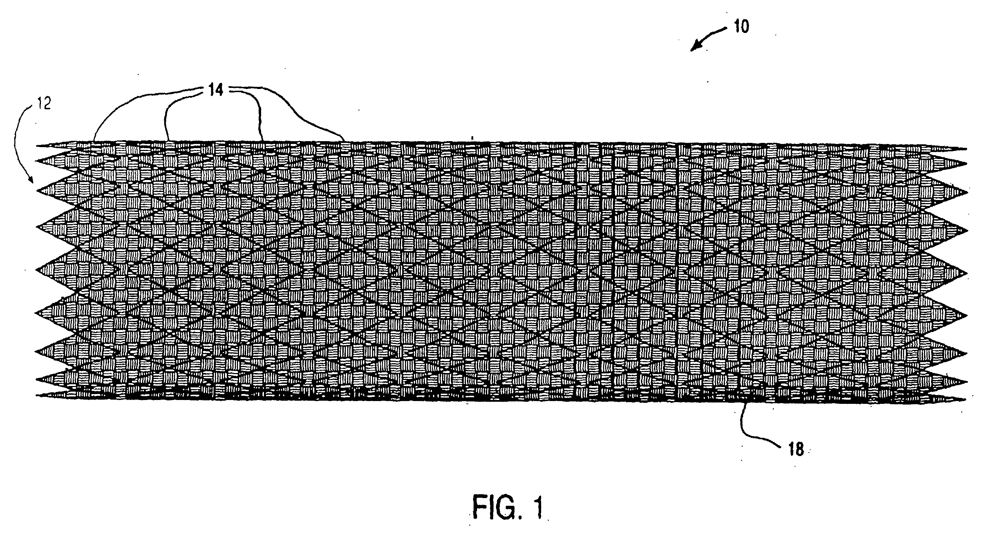

An exemplary cylindrical graft structure 10 is illustrated in Fig. 1. Prosthesis 10 comprises a perforate tubular frame 12, which here includes a plurality of independent (non-connected) ring frames 14. The tubular frame 12 supports an inner liner 18. Optionally, an outer liner is disposed over the ring frames, either instead of inner liner 18, or in combination therewith.

To secure ring frames 14 in place, and to secure the liner to the perforate tubular frame 12, the liner is typically sutured to the frame. A wide variety of alternative liner/frame attachment mechanisms are available, including adhesive bonding, heat welding, ultrasonic welding, and the like. Where inner and outer liners are used, the ring frames may be sandwiched between the liners and held in place by attaching the liners to each other.

The prosthesis 10 will typically have a length in the range from about 20 mm to 500 mm, preferably from 50 mm to 200 mm, with a relaxed diameter in the range from about 4 mm to 45 mm, preferably being in the range from 5 mm to 38 mm. Alternative stent-graft structures, including advantageous modular prostheses which may be assembled *in situ*, are more fully described in U.S. Patent Application Serial No. 08/538,706, filed October 3, 1995, and in U.S. Patent Application Serial No. 08/615, 697, filed March 13, 1996.

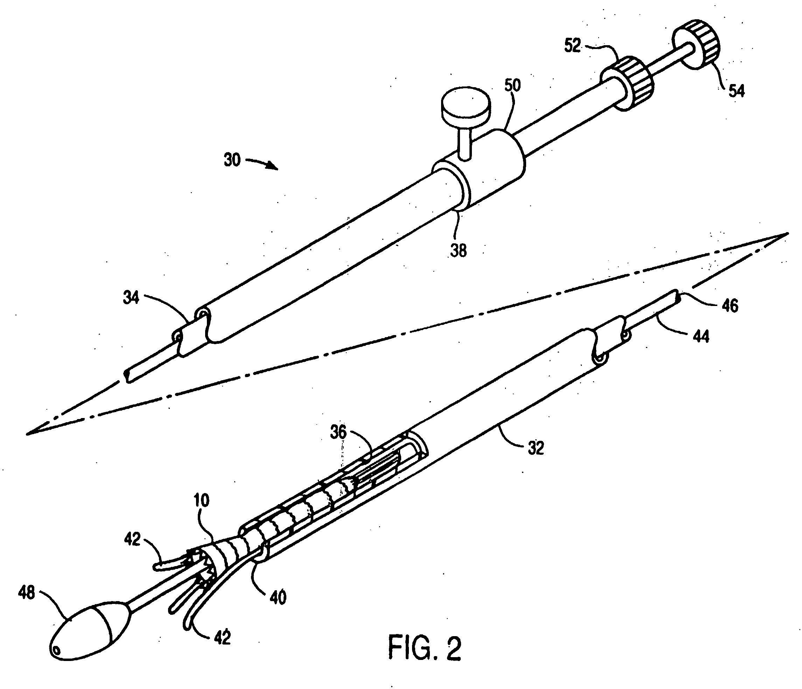

Referring now to Fig. 2, an exemplary delivery catheter 30 for use with the endoluminal prostheses of the present invention comprises a tubular cover 32 and a shaft 34. Cover 32 has a central lumen 36 extending from a proximal end 38 to a distal end 40. Shaft 34 is slidably received within central lumen 36 and extends proximally of cover 32.

A plurality of runners 42 extend distally from shaft 34. Runners 42 line a portion of the inner surface of lumen 36, and slide within the lumen with the shaft. Shaft 34 also has a lumen, in which a core shaft 44 is slidably disposed. Core shaft 44 has a guide wire lumen 46. Nosecone 48 is fixed to the distal end of core shaft 44, and can optionally be manipulated independently of runners 42.

Prosthesis 10 is radially compressed and restrained within the plurality of runners 42. In turn, cover 32 prevents runners 42 from expanding outward. Runners 42 are formed from a hard material, and distribute the expansion load of prosthesis 10 over the inner surface of central lumen 36. The deploying force is applied proximally against a slider 50 attached to distal end 38 of cover 30, while holding a luer fitting 52 at the distal end of shaft 34, thereby withdrawing the cover proximally from over the prosthesis. An additional luer adaptor 54 at the distal end of core shaft 44 allows the core shaft to be manipulated independently, and to be releasably secured to the shaft 34. Exemplary methods and devices for placement of the prostheses of the present invention are more fully described in co-pending U.S. Patent Application Serial No. 08/475,200, filed June 7, 1995.

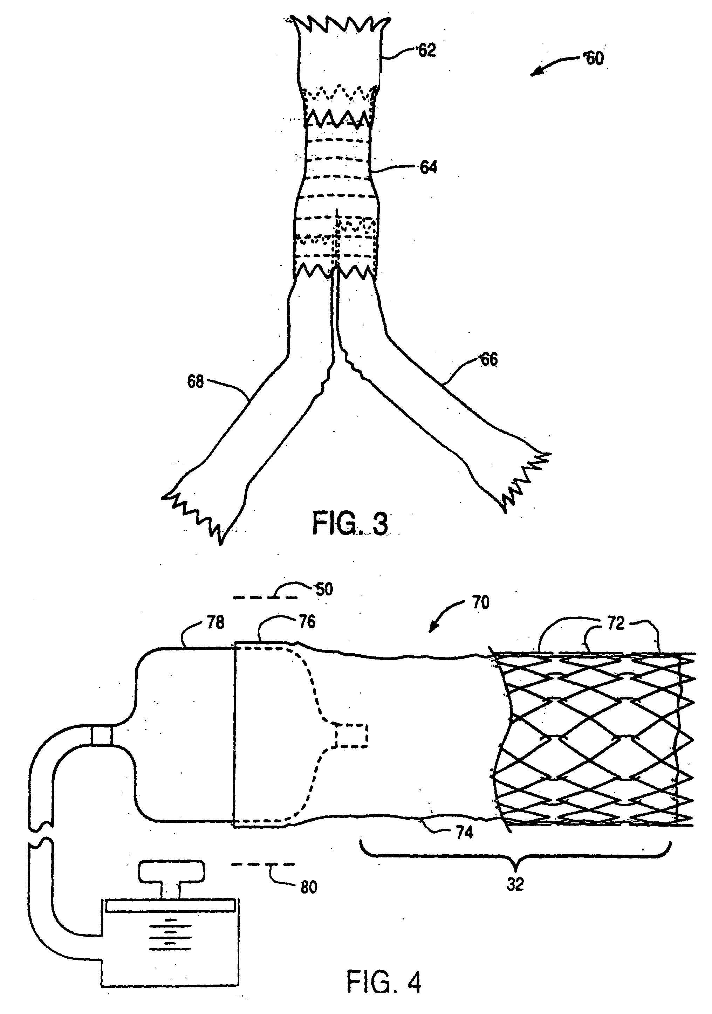

Although the structures of the present invention will generally be described with reference to simple tubular prostheses having a single lumen, it should be understood that the structures of the present invention also encompass more complex branching and modular endoluminal prostheses. Referring to Fig. 3, for example, a branching endoluminal stent-graft 60 is assembled from prosthetic modules selected to match the needs of the diseased vascular system of the patient. A common lumen cuffed prosthetic module 62 seals and anchors the assembled prosthesis in the body lumen, typically within the abdominal aorta below the renal arteries and above the left and right iliac arteries. Y-connector module 64 engages cuffed common lumen module 62, and separates the blood flow for the iliac arteries. First angled branching prosthetic module 66 and second angled branching prosthetic module 68 engage the branch lumens of Y-connector module 64 to direct the luminal flow along first and second branching body lumens.

The modular construction and expansible structure of branching prosthesis 60 allows individual tailoring of the common lumen, first branch lumen, and second branch lumen to match the geometry of the body lumen system within predetermined limits. For example, a maximum perimeter of common lumen cuffed module 62 may be selected independently of the branching lumen perimeter limits. Modular bifurcated prostheses are more fully explained in co-pending U.S. Provisional Patent Application Serial No. 60/008,254, filed December 1, 1995.

A method for expanding the prostheses of the present invention is schematically shown in Fig. 4. An expansible prosthesis 70 has frame rings 72 sutured to an expansible liner 74. Expansible liner 74 is formed from a material which expands when subjected to a stress beyond a yield strength, and which remains expanded when the stress is removed, often exhibiting little or no spring back. Alternatively, the liner may expand with some resilience, so that the liner remains taut, with no slack from overexpansion to occlude the lumen. By subjecting a cuff 76 to the expansive force of balloon 78, the liner perimeter at a selected cross-section is increased. Advantageously, the expansion of expansible prosthesis 70 may be performed prior to shipping the prosthesis as a production step, at the surgical site prior to introduction of the prosthesis within the patient body, or after deployment of the prosthesis within a body lumen using an angioplasty-type balloon catheter or other minimally invasive expansion device.

Additional benefits can be realized by the application of radially expansive force from a balloon upon the liner of a deployed prosthesis. A balloon can be used to ensure full expansion of the liner from its compressed configuration, even when an inexpansible liner is supported by a self-expanding stent. The balloon may be inflated at one or more selected locations along the prosthesis, or may alternatively be sequentially applied along substantially the entire prosthetic lumen. Balloon expansion is particularly beneficial for smoothing wrinkles in the liner (or in the entire prosthesis), especially for ensuring that externally supported stent-grafts present a smooth prosthetic lumen in endovascular applications.

Frame rings 72 of expansible prosthesis 70 may comprise a material which is resilient, malleable, or some combination of the two. When resilient, frame rings 72 will preferably be radially restrained by expansible liner 74, even after expansion of the liner to the predetermined limit. Such a liner-restrained stent-graft structure avoids any loosening of the fabric after balloon 78 has been removed.

The cuff 76 of expansible prosthesis 70 will expand to a predetermined limit, here shown as a maximum diameter 80. The expansion of expansible prosthesis 70 is generally then limited by a structural element of the prosthesis itself. In particular, once expanded to maximum diameter 80, an element of either the liner 74 or the frame rings 72, or in some embodiments, the interface between the two, impedes further expansion.

Advantageously, some portion of expansible prosthesis 70 may include more conventional inexpansible liner materials, thereby ensuring that the prosthesis is not inadvertently expanded over a preselected region. In some embodiments, an inexpansible prosthesis conduit portion 82 prevents distention of the body lumen over much of its length, with sealing between the prosthetic lumen end and the surrounding body lumen provided by an integral expansible cuff 76. Alternatively, the expansible cuff could be formed as a separate structure or module, and assembled before deployment or *in situ.*

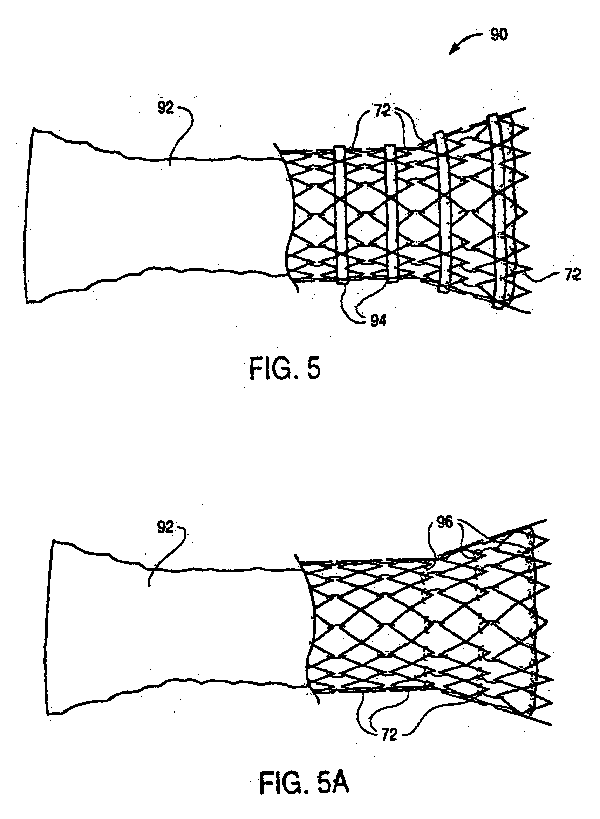

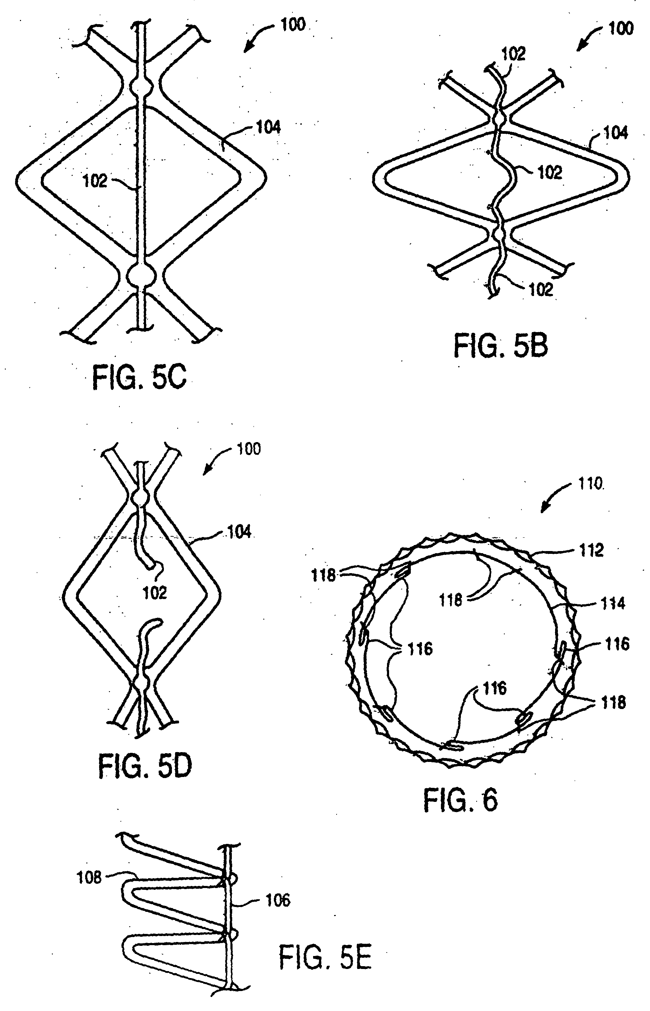

Referring now to Fig. 5, a frame belt limited stent-graft 90 includes an expansible liner 92 supported by a plurality of frame rings 72. A flexible frame belt 94 is woven through each ring frame, thereby reinforcing the frame against radial expansion when the frame perimeter is roughly equal to the circumference of the belt. Each frame belt is preferably flexible to facilitate radial compression of the prosthesis during positioning, and to adapt to expanded perimeters which are smaller than the expansion limit. Generally, the belts comprise a high strength inexpansible material so as to provide an ultimate expansion limit. Alternatively, the belts may be frangible or expansible, and thereby provide an intermediate limit, allowing still further expansion of the stent-graft.

Frame belts 94 typically comprise medical tapes or belts which are woven through or wrapped around the perimeter of the frame, optionally being disposed only at selected axial regions of the prosthesis. In some embodiments, the frame belts may be formed as integral frame elements which straighten into circumferential bands when the frame is fully expanded. Alternatively, the frame belts may comprise circumferential loops of suture 96, as seen in Fig. 5A. Such suture belts may also be used to attach the frame to the liner, either as the sole means of attachment, or in combination with some additional attachment mechanism.

Referring now to Figs 5B-D, an alternative element for restraining expansion of the stent-graft comprises a reinforced perforate frame 100 having frangible perforation reinforcement elements 102. These flexible reinforcement elements straighten and support an associated perforation dimension, here being the circumferentially oriented diagonal of diamond element 104. Such tensioned frangible elements generally comprises metal or polymer fibers which are adhesively bonded, welded, tied, riveted, integrally formed with, or otherwise attached to the frame. Perforation limiting elements might alternatively be used to limit the expansion angle of frame arms, or may take the form of compression members which restrict the axial diagonal of diamond element 104.

Reinforcement elements 102 fail in tension when the prosthesis is under a predetermined expansion threshold load, thereby allowing overexpansion of the frame beyond the predetermined limit. Alternative expansible perforation reinforcement element 106, shown as fiber tied to a zig-zag frame ring 108 in Fig. 5E, comprises an expansible material such as partially oriented yarn, and thus allows gradual overexpansion without leaving detached reinforcement element ends when an expansive load greater than a threshold load is applied.

Liners for frame limited prostheses are generally expansible to match the expanding frame, the liners typically comprising PTFE or partially oriented yarn. Alternatively, materials which are otherwise inexpansible may form expansible liners by including one or more folds in the fabric across the desired direction of expansion, as seen in Fig. 6. In other embodiments, the liner comprises an elastic tubular membrane, typically comprising spandex or urethane, which is held open by attachment to the expanded frame. Additional advantageous liner materials for frame limited expansion stent-grafts will also be described below regarding liner limited expansion.

Referring now to Fig. 6, a first embodiment of liner controlled expansion stent-graft 110 includes a frame 112 and a folded liner 114 having a plurality of axially folds 116. These folds are releasably maintained by frangible sutures 118, which separate and allow the diameter to increase incrementally. Alternatively, expansible sutures or attachments may provide gradual (rather than incremental) expansion of the liner. Advantageously, such a liner substantially retains its original porosity and strength when fully expanded.

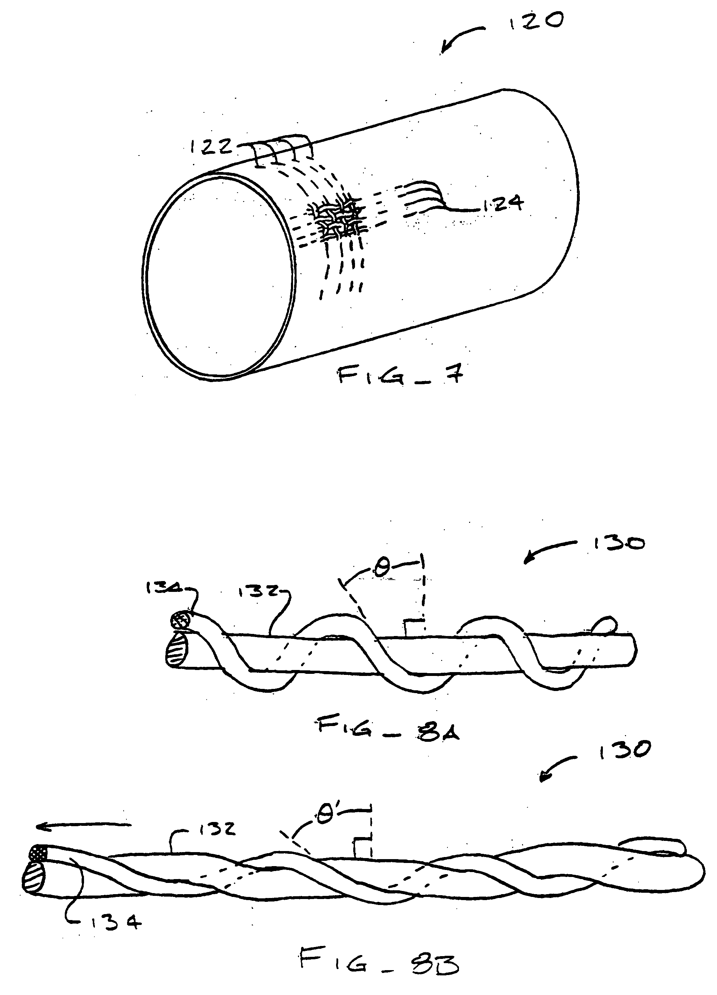

An expansible tubular vascular graft 120 is shown in isolation in Fig. 7. As used, herein, "graft" refers to structures which provide prosthetic lumens, both endoluminal structures and structures which are implanted through traditional invasive surgery. The term "liner" more narrowly refers to the prosthetic lumenal membrane of endoluminal structures. Graft 120 will generally be affixed to a radially expansible tubular frame for endoluminal use.

Graft 120 comprises a continuously woven tube with fill fibers 122 in the circumferential direction and warp fibers 124 in the axial direction. Grafts may alternatively be knitted, braided, or sewn from flat sheet material, with woven grafts generally being preferred for endovascular applications because of their compressibility and dimensional stability.

Known endovascular grafts typically comprise fully drawn polyester warp and fill fibers, often comprising a woven polyester such as Dacron™. These woven fibers provide long lasting inexpansible liners which are biocompatible, and which also promote advantageous tissue ingrowth. However, such fully drawn warp fibers are not, by themselves, expansible in situ to match a surrounding body lumen. Known liners of fully drawn polyester yarn generally provide only about 10% radial expansion before failure.

Fabric tubes woven from partially oriented yarns are extremely expansible, allowing prosthetic diameters to be increased by 80% or more. Unfortunately, expansion of much less than this amount (roughly 50% in test samples) results in an undesirable increase in porosity. Furthermore, as described above, fabrics woven with unreinforced partially oriented yarns may be susceptible to bulges or aneurysms of the prosthetic lumen during sizing or expansion. It should also be recognized that uneven expansion of such grafts by the balloon catheter or other expansion device could result in increased local porosity or open fistulas over a limited portion of the prosthetic lumen, even though the total perimeter of the graft has not been increased beyond the desired limit.

Sealing of the prosthesis against the surrounding vessel wall may also be compromised by such bulges. In particular, bulges in sealing cuffs (or otherwise adjacent to an end of the prosthesis) may result in folds that allow leakage around the prostheses, thereby maintaining pressure on the weakened portion of the vessel wall. For these reasons, graft 120 preferably includes alternative circumferentially oriented fibers which provide a limited amount of elongation.

A particularly advantageous expansible composite fiber 130 for use in grafts will be described with reference to Figs. 8A and B. Composite fiber 130 includes a core fiber 132 around which is wrapped an inexpansible fiber 134. When in a relaxed state, inexpansible fiber 134 assumes a serpentine shape, specifically being helically coiled about core fiber 132 with a pitch angle theta. It will be understood that the core fiber need not be entirely straight, but may also assume a serpentine shape when at rest. Regardless, when the fiber is axially tensioned, as shown in Fig. 8B, the length of composite fiber 130 increases, even if inexpansible fiber 134 does not increase substantially in length. This elongation of the composite fiber results, at least in part, from a change in the helical shape of the inelastic coil. In particular, the pitch angle of inexpansible fiber 134 increases to theta', and the helical diameter may also decrease, depending on the core fiber's characteristics.

Theoretically, the tensioned inexpansible fiber may eventually assume a linear shape, with the "core" fiber forced to assume a helical shape therearound. The actual elongation limit may occur substantially before that point, as the inelastic fiber will gradually absorb the tension load. It should also be understood that even an inexpansible fiber will generally increase somewhat under tension, and that even when "straightened" by tension, the composite fiber will still curve to follow the circumference of the graft, and may also bend according to the weave, knit, braid, or other circumferential fiber pattern. Nonetheless, the serpentine shape of inexpansible fiber 134 will become straighter as the graft is expanded.

Core fiber 132 may comprise any of a variety of alternative materials. Clearly, an expansible material, such as a partially oriented fiber, will provide a controlled linear expansion of the composite fiber until any tensile load beyond the yield strength of the core is substantially transferred to the inelastic fiber. Where such an expansible core is desired, the core fiber preferably comprises polyester partially oriented fiber, ideally between 40 and 120 denier. The associated wrapped inelastic fiber will typically comprise fully drawn polyester between 10 and 80 denier.

Alternatively, composite fibers may include a core fiber 132 which has a preselected low ultimate tensile strength. Such a core fiber allows expansion of the composite fiber by failing at some threshold force, the core fiber generally failing intermittently along the composite fiber. The associated inexpansible element is then free to elongate, so that the total length of the composite fiber increases.

Still further alternative core fiber materials may be used within the scope of the present invention, including those materials described hereinbelow for use as the circumferential fibers in the graft of Fig. 7. For example, fibers comprising collagen or natural wool proteins may be used in the core, or a hydrogel having sufficient mechanical integrity when hydrated. Other monofilament cores may also be used. A core fiber comprising a temperature-sensitive polymer, would allow expansion with a heated balloon within the limit of the wrapped inelastic material. Preferably, the transition temperature is above body temperature so that such a heat sensitive polymer composite fiber is resistant to creeping when subjected to ordinary stresses at body temperatures. Hence, the composite fiber of the present invention encompasses a wide range of alternative materials.

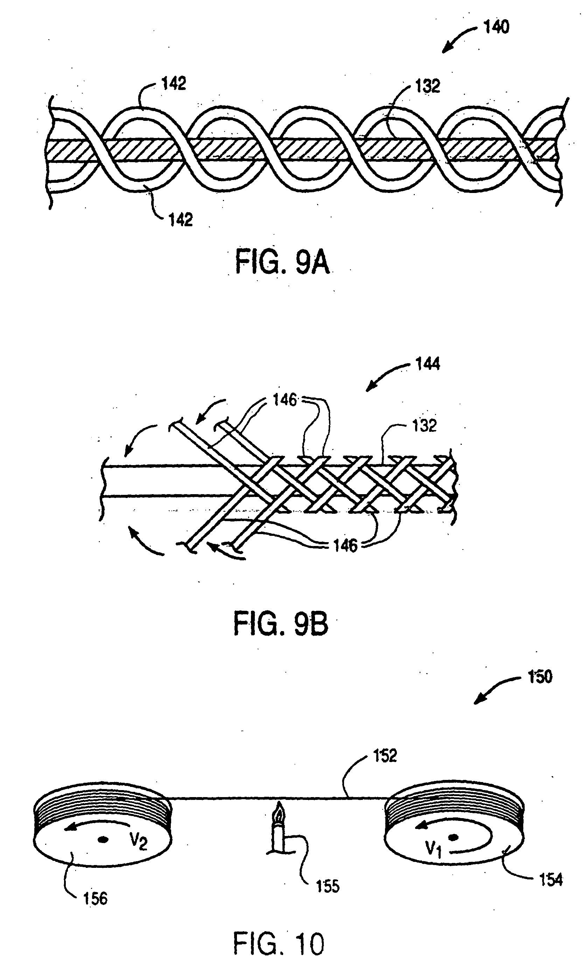

Alternative composite fiber wrap structures are shown in Figs. 9A and B. A first alternative composite fiber 140 includes a plurality of inexpansible fibers 142 wrapped over core fiber 132 in the same direction. A second alternative composite fiber 144 has a plurality of inexpansible fibers 146 counterwound over the core. As seen in Fig. 9B, such counterwound fibers are optionally braided over the core, which will minimize changes to the core's path when tension is applied. A composite fiber having a core wrapped with two counterwound inelastic fibers would minimised the cost and complexity of preparing a counterwound composite fiber.

An alternative circumferential fiber which will provide controlled radial expansion of the graft of Fig. 7 comprises fully oriented yarns, typically of polyester, which have been annealed at less than melting temperature. The initial fully oriented yarn is widely available, being commonly used in knitting and weaving. The yarn is generally allowed to decrease in length during the annealing process, optionally by use of a collapsible cone, or by use of a reel system 150 as illustrated in Fig. 10.

Reel system 150 is similar to a drawing systems used to orient yarns. Fully oriented yarn 152 is supplied by a source reel 154 at a relatively high linear velocity V1. The yarn is heated (as represented by heating element 155), generally to a temperature of less than the melting point (250°C to 260°C for Dacron™), preferably to a temperature of about 150°C to 260°C. The heated yarn is allowed to shrink, and is loaded on a take-up reel at a lower linear velocity V2.

With proper heat treatment temperatures, time, and shrinkage, grafts having annealed fully oriented yarns in the fill could provide controlled expansions of the prosthesis in the range from about 25 to 60 percent. The annealed fully oriented yarn begins to yield plastically at a substantially lower load level than the untreated fully oriented yarn. Once elongation begins, the yarn can be controllably elongated with increasing force. Advantageously, once the yarn is expanded within the structure of the graft, the properties of the yarn will gradually return to the properties of the pre-heat-treated fully oriented yarn. In fact, the maximum load at failure (in grams per denier) is similar to untreated fully oriented yarns, as long as the same base polymer is used in both.

A further alternative circumferential fiber which will provide controlled radial expansion of the graft of Fig. 7 comprises fully drawn yarns, typically of polyester, which have been texturized by twisting the yarns first in one direction, and then in the other, and by heat setting the yarn while it is twisted. A particularly advantageous texturizing process is sometimes referred to in the textile industry as "high crimp retention texturization, " and is commercially available through Becker Industries. Yarns texturized with temperatures between about 200°C and 220°C and with spindle speeds of between about 4,200 and 4,400 RPM have produced linear elongations at break of between about 20 and 25%. The texturized yarn is preferably woven with minimum tension in the fill so that the texturized yarn would not be stretched during graft fabrication.

The texturized yarn produces a "crinkled" lumen surface, while the size of the lumen is generally smaller than would otherwise result from untreated fully oriented yarn. Upon balloon expansion, the diameter of the fabric increases to approximately the dimensions of an equivalent graft woven from fully oriented yarn. Hence, the texturized yarn in the fill provides an effective increase from the initial, unexpanded diameter. Once again, the material characteristics after expansion will be approximately equivalent to the original fully drawn yarns. Total effective expansions of up to about 20% should be available using such texturized yarns.

A still further alternative circumferential fiber which may provide controlled radial expansion of the graft of Fig. 7 comprises a temperature sensitive polymer such as a side-chain crystallizable copolymer, similar to those available from Landec Corp. of Menlo Park, California under the trademark Intelimer® , or a radiation cross-link polymer.

Side-chain crystallizable polymers provide the ability to change state, between amorphous and crystalline, at a predetermined temperature. The transition temperature and physical properties of the material can be tailored, and the material is now being applied to medical products.

For use in the fill of the controlled expansion graft of the present invention, a fiber comprising a side-chain crystallizable polymer would preferably transition from inexpansible at body temperature to expansible when heated to somewhat above body temperature, i.e., about 43°C. To expand the graft *in situ* beyond the size allowed by the normally inexpansible fill fibers, a balloon would be inserted within the graft lumen and inflated with saline solution (or some other medium) which had been heated to a temperature slightly, above the transition temperature. The balloon would warm the fill fibers and the side-chain crystallizable polymer to above the transition temperature, allowing the fill fibers to elongate. Expansion could then proceed from radial expansion force provided by increasing balloon pressure.

Advantageously, a prosthesis having such a temperature-sensitive graft may be introduced and positioned at the target site of a body lumen independently of the expansion balloon. A self-expanding stent-graft including a side-chain crystallizable polymer will preferably be compressible to a narrow diameter configuration, without any increase in delivery catheter diameter required to accommodate a concentric balloon. Alternatively, concentric prosthesis/expansion balloon delivery systems are also feasible.

Once the side-chain crystallizable polymer fill fibers were expanded to conform the anatomy of the vessel, the balloon would be deflated so that the graft would again cool to body temperature, at which the fill fibers would resume their more crystalline state. Optionally, cooler saline solution could be infused in the balloon to reduce the graft temperature before deflation. The general properties and capabilities of side-chain crystallizable polymers were described in *Temperature Compensating films* for *Produce,* **Prepared Foods** (September, 1992). As is generally true , the axial fibers may comprise more conventional graft materials, such as polyester or PTFE.

Radiation cross-link polymers behave very differently, generally shrinking when heated above a certain temperature. Advantageously, the reduced dimensions of these polymers may then be set by cooling. By forming oversized grafts having circumferential fibers which comprise such radiation cross-linked polymers, preferably having a shrinkage temperature above body temperature, the graft cross-section could be controllably fitted by radially shrinking of the graft *in situ* over a heated balloon. Once again, infusion of cooler fluid will then set the prosthesis lumen size. Endoluminal prosthesis having such a shrink-to-fit graft structure will benefit from a high compressibility and flexibility to facilitate manipulation of the oversized graft within the body lumen prior to shrinking.

A still further alternative circumferential fiber which may provide controlled radial expansion of the graft of Fig. 7 comprises a hydrogel or other monofilament. Hydrogels are hydrophilic polymers that absorb water, thereby changing mechanical properties. Fibers comprising hydrogels would generally become softer and more extensible when placed in the aqueous environment of the body.

Hydrogels could be crosslinked or co-polymerized with hydrophobic monomers to maintain the designed mechanical integrity when hydrated. The intermolecular interactions of hydrogels can be broken, thereby allowing the hydrogels to change shape, by heating or by the action of certain organic solvents. Hence, fill fibers which comprise hydrogels may be controllably expanded. These properties of Hydrogels are more fully described in *Thermoplastic Hydrogels,* 23 **British Polymer Journal**, pages 257-259 (1990).

Still further alternative materials may provide fill fibers having the desired controlled radial expansion, including PTFE, Collagen, and natural wool proteins. PTFE is an expansible, biocompatible material which has an extensive history of use in the medical field. Similarly, collagen, which is available in a number of forms from Collagen Corp. of Palo Alto, California, is a biocompatible material which has been used in medical applications.

Referring back to Figs. 3 and 4, two particularly advantageous applications for expansible materials in endoluminal prostheses may generally be classified as integral sealing cuffs, such as cuff 76, and separate sealing cuffs, such as common lumen cuffed prosthetic module 62.

As described above, the selective plastic expansion of one or more ends of the prosthesis promotes sealing between the prosthesis and the surrounding body lumen without distending the body lumen along the entire prosthetic lumen. Advantageously, prostheses having such sealing cuffs thereby provide therapy for an aneurysm which minimizes stress on the weakened portion of the vessel wall. Integral and separate sealing cuffs which include the expansion limiting structures described above will further help to limit the distention of the healthy portion of the body lumen against which the prosthesis is sealed.

Integral sealing cuffs are often formed as an extension of the prosthetic liner, optionally by a simple change in the fill material of the liner, the structure otherwise being consistent with the body of the prosthesis. A particularly advantageous integrated sealing cuff may be formed by switching from an inexpansible fully drawn fill material to a partially oriented yarn adjacent the end of the liner.

Alternatively, the cuff may have a structure which produces very different expansion characteristics than the remaining body of the prosthesis. Much of expansible prosthesis 70, for example, comprises inexpansible prosthesis conduit portion 82. Within a weakened blood vessel, this conduit provides a flow path for the blood. While this conduit preferably flexes with the body lumen, the axial length of conduit portion 82 will preferably substantially remain constant, particularly while the prosthesis increases in cross-section during deployment (or any size adjustment) to ensure that the conduit extends beyond the diseased portion of the body lumen. On the other hand, the sealing cuff serves primarily to seal between the conduit and the surrounding body lumen, and optionally to help anchor the prosthesis. Hence, changes in the axial length of the sealing cuff are of less concern.

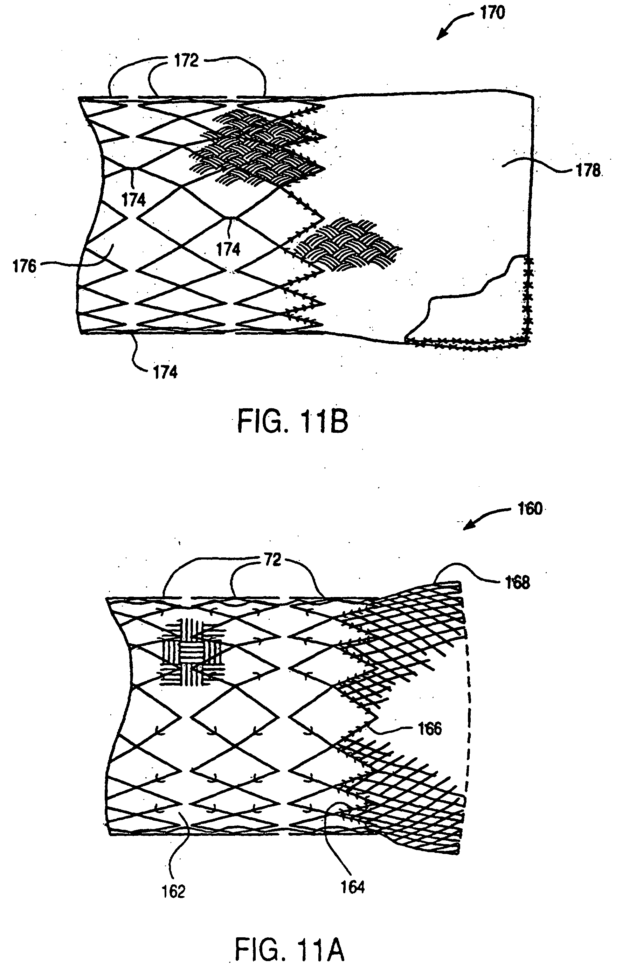

Referring now to Fig. 11A, an integral braided cuff prosthesis 160 comprises a plurality of independent ring frames supporting a conventional liner 162. An axial end 164 of the liner is stitched to the adjacent ring frame with a crown stitch pattern 166, which also attaches a braided cuff 168. Alternatively, the braid may be stitched along the midline of the adjacent frame ring and extend equidistant inward along liner 162. Braided materials are highly radially expansible, but are not often used for prosthetic conduits as radial expansion generally also results in a reduction in length of the prosthetic lumen. However, as this coupling of the radial and axial dimensions does not impeded the sealing of the end of the prosthesis, braided cuff 168 is free to shorten as required. In fact, the cuff may alternatively be oriented back along the prosthesis.

Referring now to Fig. 11B, an alternative integral braided cuff prosthesis 170 comprises interconnected ring frames 172 having joints 174 which maintain the axial length of the prosthetic conduit during radial expansion. The liner 176 comprises a braided material which extends axially beyond the frame to also form a folded cuff 178. The braided material is folded outward and extends back to the frame, where it is attached by crown stitching. Hence, as the prosthesis expands radially, the length of the cuff decreases, but the length of the prosthetic conduit is not changed. Optionally, expansion of the frame may be limited as described above.

Separate sealing cuffs may make use of similar structures, often having a structure which limits a cross-section of the cuff along an interface with the remainder of the prosthesis. Use of such an inexpansible interface in common lumen cuffed prosthetic module 62 (Fig. 3) facilitates safe expansion of Y-connector module 64 therein during assembly. Alternatively, a separate sealing cuff which extends axially from within an inexpansible prosthesis may rely on that other prosthesis to limit expansion.

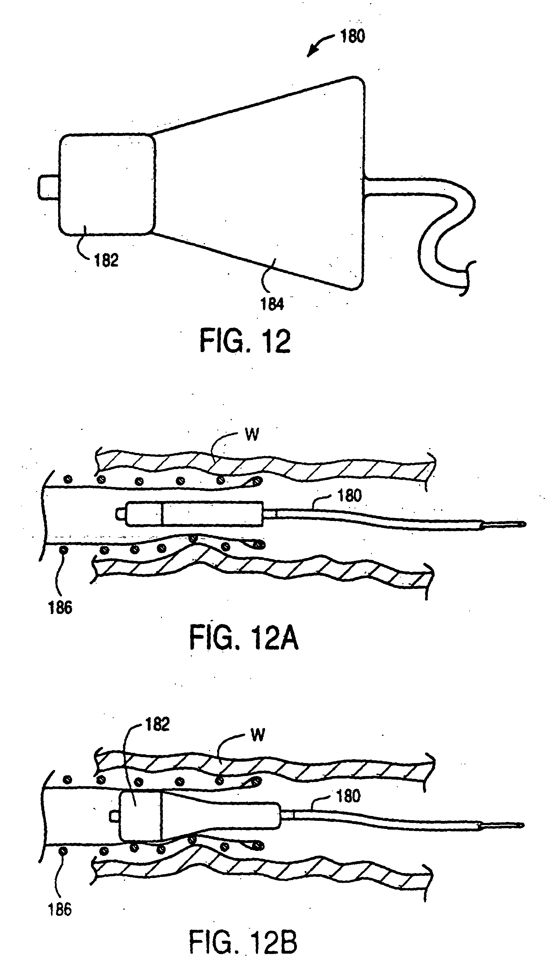

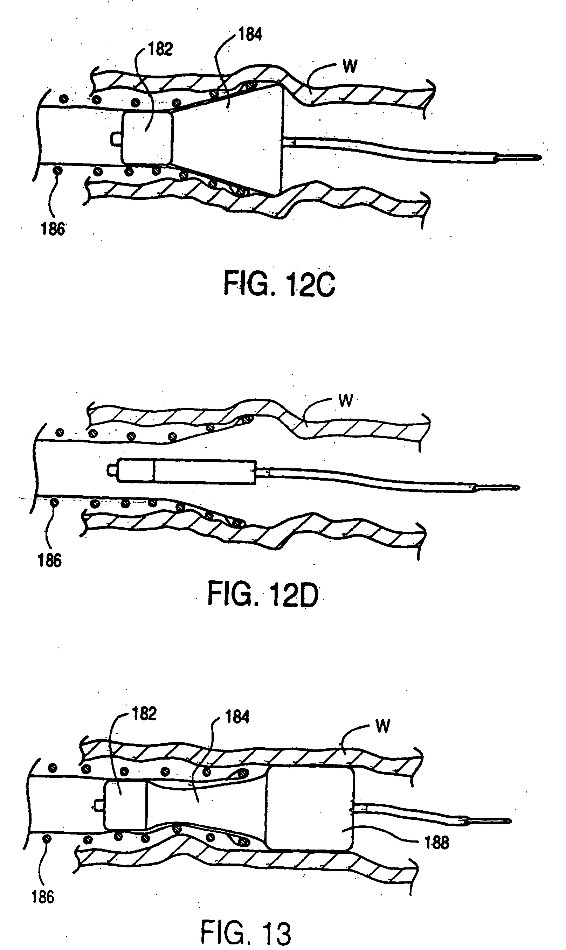

Referring now to Figs. 12-13, an advantageous balloon catheter 180 facilitates selective expansion of an integral or separate sealing cuff within a body lumen without the prosthesis sliding off the end of the balloon. Balloon catheter 180 comprises a prosthesis gripping chamber 182 and an expansion chamber 184. The balloon is inserted into a body lumen and positioned within a prosthesis 186. Prosthesis gripping chamber 182 is first inflated, optionally along an inexpansible portion of the prosthesis, to firmly engage the prosthesis and prevent relative movement between the prosthesis and balloon.

The expansion chamber 184 is then inflated to expand the end of prosthesis 186 to the desired cross section, after which both chambers can be deflated and the balloon removed. Optionally, a vessel wall gripping chamber 188 may be inflated prior to expansion with the expansion chamber, thereby securing the position of prosthesis and balloon with respect to vessel wall W, as can be seen in Fig. 13. The vessel wall gripping balloon is preferably expandable using sufficiently low pressures to avoid injury to the vessel while engaged.

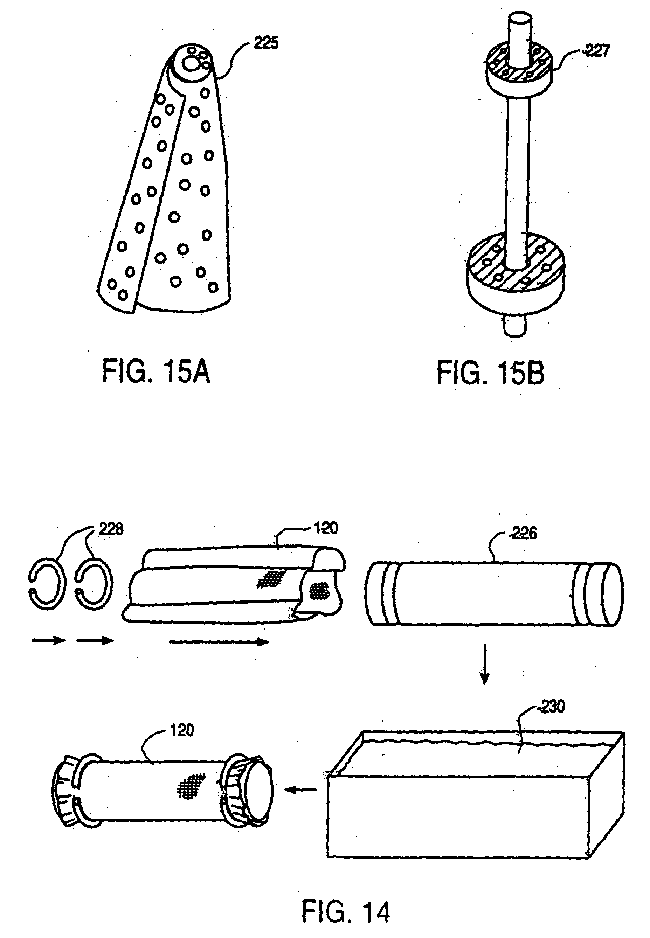

The present invention provides still further materials to allow expansion of graft 120 of Fig. 7. Referring now to Fig. 14, graft 120 is chemically compacted by supporting the graft on a mandrel 226 with rings 228, and immersing the graft in a compaction solution 230. Work in connection with the present invention has shown that the expansibility of fully drawn polyester liners can be enhanced by such chemical compaction. Compaction appears to enhance the expansibility of the fibers by forming amorphous zones within the crystalline structure of the fully drawn polymer. Expansion may be plastic, resilient, or some combination of both.

Compaction of polyester also swells the fibers and decreases their length, and has therefore been used to limit the porosity of braided or knitted surgical vascular grafts, for which an increase in the total volume of graft material is not a concern, but which tend to otherwise be quite porous. Although an increase in liner volume also increases the compressed size of endoluminal prostheses, and is therefore potentially deleterious for intravascular maneuvering, the beneficial, controlled expansibility provided by such a compacted, fully oriented material more than compensates for the increase in volume. Moreover, the ability to expand the prosthesis *in situ* may substantially mitigate any swelling of liner thickness by allowing the use of a prosthesis having a smaller initial cross-section.

For these reasons, fill fibers 122 of graft 120 may optionally comprise a fully drawn polymer, typically being a polyester, and ideally being a polyester yarn such as Dacron^{TM} type 56, commercially available from Dupont. Surprisingly, radial expansibility for a given strength may be enhanced by weaving fill yarns which are heavier than the yarns of the adjacent warp fibers 124, particularly when such heavy yarns are combined with the loose weave described hereinbelow. Hence, the fill yarns will generally be at least about 60 Denier, ideally being about 80 Denier, while the warp yarns are preferably finer, ideally being about 40 Denier.

When graft 120 comprises a compacted, fully drawn polymer fibers, radial expansibility can also be enhanced by a looser weave, the warp preferably being relatively sparsely woven with the fill. In other words, a relatively high number of axial elements per inch (generally referred to as "ends per inch") are preferably woven for the given number of fill yarns per inch (generally referred to as the "pick count"). Preferably, the liner will comprise less than about 160 ends per inch, ideally, about 150 ends per inch, and a fairly standard pick count of about 78. Alternatively, expansibility may also be enhanced by a looser weave in which the fill is relatively sparsely woven with the warp, or by some combination of the two. Excessively loose weaves, however, may increase porosity, eventually resulting in excessive blood loss through the liner.

Compaction solution 230 may comprise a variety of alternative compounds, and may be gaseous or liquid, as is generally described in the patent literature. One group of compaction solutions comprises acidic organic compounds, preferably with a halogenated aliphatic hydrocarbon of up to 6 carbon atoms. A particularly preferred compaction solution comprises between about 92% and 100% methylene chloride with between about 0% and 6% hexafluoro 2-propanol. Alternative compaction solutions include NO₂, either in the gaseous phase or in solution. In some embodiments, compaction can even be accomplished in a steam atmosphere.

The duration of the compaction bath or exposure will depend on the specific compaction compounds and conditions, and possibly on the weight of the fibers being compacted. When using the preferred compaction solution of methylene chloride with hexafluoro 2-propanol, fill fibers 122 will preferably be compacted for between about 1 and 10 minutes, with the ideal compaction time dependent on the selected compaction method.

Additional variables of the compaction process may also control the expansibility of graft 120. For example, the amount of shrinkage of the fill fibers may be limited by the size of mandrel 226 relative to the pre-compacted cross-section of the liner. Where the fibers are compacted after the liner is formed, as shown in Fig. 14, the axial shrinkage may be independently varied by selective positioning clips 228 along the liner material. Variations in the bath temperature may also influence expansibility. The liner will typically be washed after compaction to remove any potentially harmful compaction solutions.

Generally, compaction of fully drawn polyester fill fibers will provide radial expansibility of at least about 15%. Preferably, the liner will be radially expansible by about 20% or more, ideally being radially expansible by at least about 30%. In most cases, the liner will preferably have a porosity of less than about 1000 ml/min/cm² after expansion, porosity typically being measured by water permeability at a pressure of 120 mm of mercury. As an expansile graft expands radially, porosity increases. A porosity range from about 500 ml/min/cm2 to 2000 ml/min/cm2 typically coincides with a 45% expansion of the graft's diameter.

As described above, it may be advantageous to loosely weave the liner to enhance expansibility. Hence, as compaction can subsequently reduce porosity below the limit given above, it is often desirable to initially weave the liner with a higher initial porosity prior to compaction. Generally, the liner can be fully expanded using an internal expansion balloon with up to about 4 atmospheres, but will not elongate when subjected to physiological loads such as blood pressure.

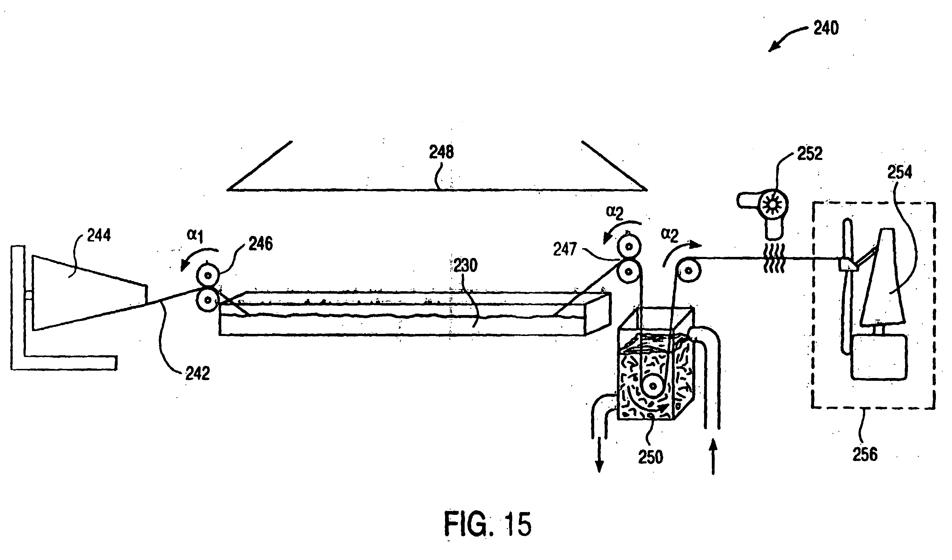

An alternative method for compacting fully drawn polyester fibers will be described with reference to Fig. 15. As explained above, radial expansibility is highly advantageous for liners of endoluminal prostheses. In contrast, it may be desirable to minimize axial expansibility of grafts, or to limit such axial expansibility to selected regions. Furthermore, it may be beneficial to avoid compaction of liner fibers which do need not to be expansible. Toward that end, graft 120 may be selectively woven from fibers which have previously been compacted, generally as fill fibers 120, the warp fibers being inexpansible, typically fully drawn polyester, thereby providing radial expansibility without axial expansibility.

Fig. 15 schematically illustrates a system for compacting fibers 240 which takes yarn 242 from a supply spool 244 with a first drive assembly 246. The yarn is fed into compaction bath 230 at a first drive speed α₁, and is removed at a second speed α₂ by a second drive assembly 247, the removal speed generally being lower than the feed speed to accommodate shrinkage. A fume hood 248 removes fumes released from the bath. The compacted fiber is rinsed in a water bath 250 and dried by drier 252 before being wound on compacted fiber spool 254 of a textile wrapping apparatus 256. The compaction process, including the compounds in the compaction bath and the like, will generally be similar to those described above regarding Fig. 14.

Another method for providing compaction of graft material prior to forming the graft is bulk compaction of the fill element, which will be explained with reference to Figs 15A and B. In one embodiment, bulk compaction can be performed by winding polyester and submerging the polyester windings in a compaction solution. In an exemplary embodiment, the polyester is wound onto a collapsible cone 225. The collapsible cone may be formed from an overlapping coiled sheet of a material which is flexible and substantially resistant to the compaction solution, the cone ideally comprising PTFE. The polyester may be wound on collapsible cone 225 while cone 225 is releasable supported by a standard textile cone. After the polyester is wound, the polyester windings are removed from the textile cone with collapsible cone 225, the PTFE material providing an easy transition and support for the otherwise flimsy polyester.

The polyester and collapsible cone 225 are then placed over a compaction fixture 227, which is initially smaller in diameter than the collapsible cone to allow for shrinkage. This assembly is then submersed into the compaction mixture, ideally within an ultrasonic bath (such as within an ultrasonic cleaner). An exemplary compaction solution for this method comprises between about 3 and 6% hexafluoro-2-propanol, and from about 94 to 97% methylene chloride. The ultrasonic vibrations ensure that the compaction mixture comes into contact with the polyester material throughout the depth of the windings. The polyester is often left in the bath until fully compacted, and then rinsed ultrasonically in isotonic water. The compacted polyester and its fixture are then placed into a warm oven until dry.

The dried, compacted polyester is then rewound at a low RPM onto a standard textile cone. After the compacted polyester is rewound, it can then be woven into the fill of the graft to provide radial expansion, the warp typically comprising uncompacted polyester.

Collapsible cone 225 will often be formed from a thin, pliable sheet which has been coiled into a conical shape. Holes may be cut into the sheet to enhance contact between the compaction solution and the windings. Optionally, the collapsible cone may be formed by simply cutting a sheet of PTFE to shape, and winding the sheet over a standard textile cone. When the polyester windings and collapsible cone 225 are slipped off the standard cone and imersed in the compaction solution, the polyester shrinks. To accomodate this shinkage, the PTFE sheet decreases in diameter by coiling tighter with an overlapping effect to fit the compaction fixture. Compaction fixture 227, which may be formed as a rod supporting rings with holes for the compaction fluid, surves to guide the shrinkage and support the polyester when it is rewound back onto a standard textile cone. Compaction fixture 227 will also comprise a material which is resistant to the compaction mixture, typically comprising stainless steel.

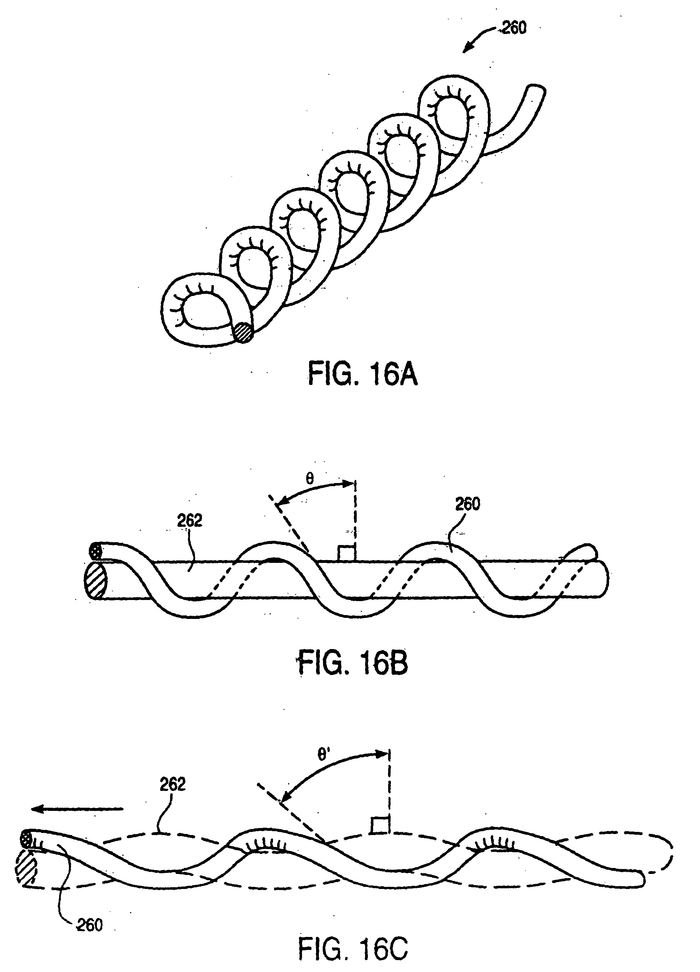

Figs. 16A-C illustrate an alternative expansible fill element for use in graft 120 of Fig. 7. A coiled yarn 260 generally comprises inexpansible fibers, typically being fully drawn polyester. Coiled yarn 260 is generally formed by wrapping a core fiber with the inexpansible fiber, and then weakening or at least partially dissolving the core fiber, either before or after forming graft 120, leaving the inexpansible fiber in the desired helical configuration. As can be understood with reference to Fig. 16C, such a helical coil allows axial expansion without relying on elongation of the fibers themselves, at least in part due to a change in the helical pitch from θ to θ'. Suitable materials for the core include nylon, collagen, and PVA, which can be dissolved or weakened without destroying a surrounding polyester wrap. Optionally, a plurality of inexpansible fibers 260 may be wrapped over core fiber 262 to provide multiple coaxial coils, as generally shown in Fig. 9A, or counterwound as shown in Fig. 9B. A preferred arrangement includes two counterwound inelastic yarns over the core. As described in the parent to this application, an expansible core fiber may alternatively allow expansion without weakening or dissolving the core.

As was also described above, an alternative circumferential fiber which will provide controlled radial expansion comprises fully oriented yarns, typically of polyester, which have been annealed at less than melting temperature (250°C to 260°C for Dacron™), preferably to a temperature of about 150°C to 260°C. It is also possible to anneal the woven graft as a whole. Advantageously, the expansibility provided by annealing and compaction may be at least partially cumulative. Hence, yarns and/or liners may be both compacted and annealed to increase expansibility. A particularly preferred combined method will comprise annealing, and then compacting, and then again annealing to maximize expansibility. Advantageously, as the annealed yarn is expanded within the structure of the graft, the properties of the yarn will gradually return to the properties of the pre-heat-treated fully oriented yarn.

As described above, loosely weaving a fabric of compacted, fully oriented fibers enhances the expansibility of the graft, but can increase porosity and leakage. The present invention recognizes that this may be an advantageous tradeoff, so long as the total leakage through the liner remains acceptable. To avoid excess porosity over the entire axial length or radial circumference of the graft, the graft of the present invention can be selectively woven with a loose, expansibility enhancing pick count (or ends per inch) over only that portion of the liner for which expansibility is particularly important, such as at the axial ends to promote circumferential sealing against a surrounding body lumen.

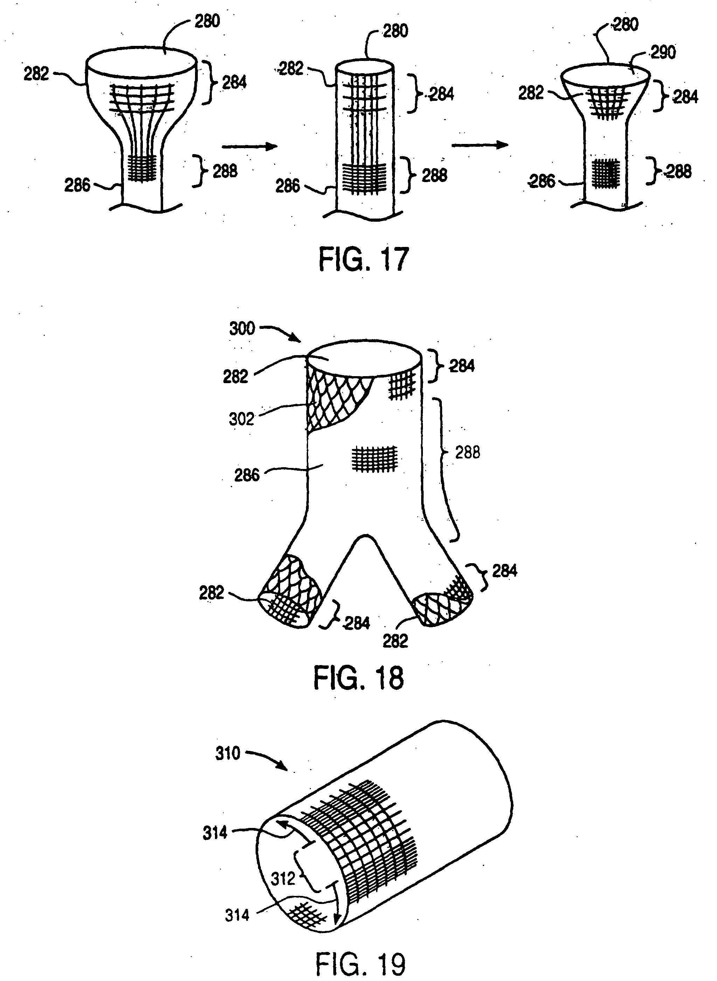

Referring now to Fig. 17, a selectively woven liner 280 includes an end portion 282 having a relatively low pick count, and a middle portion 286 with a relatively high pick count 288. Preferably, end portion 282 also has a heavier fill yarn, as explained above. The end portion is initially woven with an enlarged cross-section at the end portion, and the graft is compacted over a mandrel as illustrated in Fig. 14 to produce a uniform cross-section. The looser weave and increased shrinkage during compaction allow substantially greater expansibility of the end portion, preferably allowing over about 25% expansion, and ideally allowing over about 30% expansion. In contrast, the middle portion, which will span the aneurysm or other diseased portion of the body lumen, will have significantly less expansibility, often being less than about 10%, and will also have lower porosity, generally being less than 1000 ml/min/cm². Thus, for example, the end of the prosthesis can be expanded to a tapered profile 290 with a profiled balloon so as to promote circumferential sealing of the end of the liner against a surrounding body lumen, but without producing excessive total leakage through the liner. Typically, selectively woven liner 280 will be supported by a radially expansible frame, which has been omitted from Fig. 17 for clarity.

Referring now to Fig 18, a bifurcated prosthesis 300 having a selectively woven liner is illustrated with a portion of the bifurcated frame 302 removed for clarity. Such selectively woven liners may be used on a wide variety of prosthetic structures, including modular prostheses, integral bifurcated prostheses, trifurcated prostheses, or even straight grafts. Regardless, the integrity of the main body of the graft is maintained, while expansibility of the ends is enhanced. Not only is expansion of the ends particularly beneficial for circumferential sealing, but the ends may also be more tolerant of an increased porosity, even above about 1000 ml/min/cm², where these porosities are limited to the portion adjacent the interface between the healthy tissue and the prosthesis. In fact, the increased porosity at the ends may even help to promote tissue ingrowth at this interface.

Referring now to Fig. 19, an alternative selectively woven liner 310 includes at least one loosely woven axial zone 312, which has warp yarns with fewer ends per inch than at least one tightly woven zone. Alternative liner 310 is initially woven with a relatively large overall cross-section, and is then compacted to a smaller diameter. Subsequent radial expansion of alternative liner 310 will result in enhanced expansion along loosely woven axial one 312, while tightly woven zones 314 will maintain their low porosity so that total leakage through the graft is acceptable.

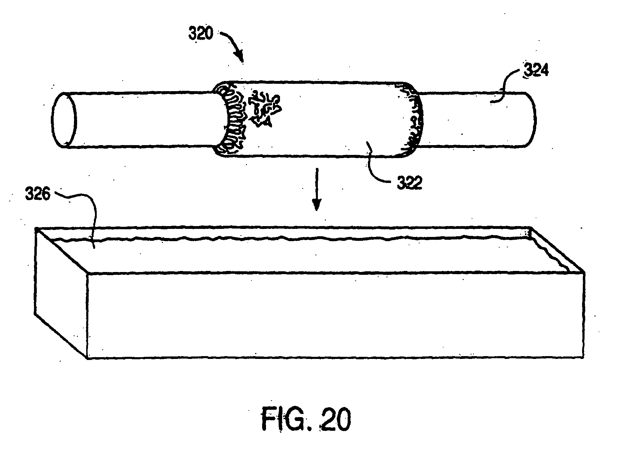

Referring now to Fig. 20, a temperature controlled prosthesis 320 can be produced very simply by forming a radially expansible fabric tube and placing the tube over a mandrel 324, the mandrel preferably having a low friction outer surface. The mandrel and tube are then immersed or otherwise coated with a temperature sensitive polymer 326, such as a side-chain crystallizable copolymer similar to those available from Landec Corp., of Menlo Park, California and sold under the trademark Intelimer® . The tube and polymer dispersion can be removed axially from the mandrel.

As described above, side-chain crystallizable polymers have the ability to change state between amorphous and crystalline at a predetermined temperature. The transition temperature and physical properties of the material can be tailored, and the material is now being applied to medical products. The temperature sensitive polymer will reinforce the liner so that the prosthesis resists changes in the lumenal cross-section at body temperatures, but will allow the prosthesis to be radially expanded, and possibly later resized, with a heated and/or cooled balloon catheter.

Specifically, temperature sensitive polymer 326 preferably comprises a side-chain crystallizable polymer which transitions from a substantially rigid crystalline state at body temperature, to an expansible amorphous state when heated to above body temperature, i.e., about 43°C. Temperature sensitive prosthesis 320 will generally be introduced and positioned in a small diameter configuration. To expand the graft in situ, saline solution (or some other medium) which had been heated to a temperature slightly above the transition temperature will raise the prosthesis temperature, typically from within a balloon inserted into the prosthetic lumen. The balloon will warm the side-chain crystallizable polymer to above its transition temperature, allowing the prosthesis to expand radially. Expansion could then proceed from radial expansion force provided by increasing balloon pressure.

Fabric tube 320 is preferable highly radially expansible, and may also be somewhat resilient to facilitate shrinking the prosthesis around a heated balloon. Hence, the tube will generally comprise a knit tube, ideally comprising a jersey knit. When rigid, the fabric tube and polymer dispersion will provide a fiber reinforced composite structure, and the fabric will also help maintain the integrity of the polymer when it is heated to an amorphous state. Optionally, the fabric tube and polymer dispersion together define and/or seal the prosthetic lumen.

Once the temperature sensitive prosthesis is expanded to conform to the anatomy of the surrounding body lumen, the balloon will be deflated so that the graft cools to body temperature. Optionally, cooler saline solution could be infused in the balloon to reduce the graft temperature before deflation.

## Claims

1. A controlled expansion endoluminal prosthesis (10) comprising:
a radially expansible tubular frame (12) having an inner surface and an outer surface; and
a liner (74) disposed on at least one of the inner and the outer surface of the frame (12);
**characterized in that**
the liner (74) is plastically expansible and at least one of the frame (12) and the liner (74) includes a reinforcing element (96) which limits radial expansion of the prosthesis (10) at a predetermined expanded size.

2. A prosthesis as claimed in claim 1, **characterized in that** the frame (12) includes a plurality of reinforcing elements (96) which restrain radial expansion of the frame (12) so that the frame (12) defines the predetermined size.

3. A prosthesis as claimed in claim 2, **characterized in that** the reinforcing elements (96) releasably restrain resilient expansion of the frame (12) at the predetermined size, the frame (12) comprising a self-expanding structure.

4. A prosthesis as claimed in claim 2, **characterized in that** the reinforcing elements (96) limit plastic expansion of the frame (12) at the predetermined size, the frame (12) comprising a plastically expansible structure.

5. A prosthesis as claimed in claim 1, **characterized in that** the reinforcing element comprises a fill element (122) of the liner so that the liner defines the predetermined expanded size.

6. A prosthesis as claimed in claim 5, **characterized in that** the fill element (122) comprises a composite yarn (130) including serpentine inexpansible fiber (134) and a second fiber (132), and wherein the inexpansible fiber straightens during expansion of the liner to the predetermined expanded size.

7. A prosthesis as claimed in claim 1, **characterized in that** the reinforcing element (102) breaks or yields when a radial expansive force exceeding a predetermined threshold force is applied, thereby allowing the prosthesis (10) to be expanded beyond the predetermined expanded size.

8. A prosthesis as claimed in claim 1, **characterized in that** said plastically expansible liner is disposed over at least a portion of the inner or outer surface of the frame (12), which comprises a circumferentially oriented yarn including fully drawn fiber which defines a maximum expanded perimeter of the liner.

9. A prosthesis as claimed in claim 8, **characterized in that** the fully drawn fiber is wound around a second fiber, and wherein the maximum perimeter is defined by straightening the fully drawn fiber.

10. A prosthesis as claimed in claim 1, **characterized in that** the plastically expansible liner comprises a fabric tube including composite yarns (130) having serpentine inexpansible fiber (134) and expansible fiber (132) so that the inexpansible fiber (134) straightens during expansion of the graft to prevent expansion of the fabric beyond a predetermined limit.

11. A prosthesis as claimed in claim 1, **characterized in that** the radially expansible tubular frame defines an axial direction and a circumferential direction; and the plastically expansible liner comprises a fabric tube which includes circumferentially oriented fiber (122) that has been chemically compacted.

12. A prosthesis as claimed in claim 11, **characterized in that** the circumferentially oriented fiber has been chemically compacted prior to forming the tube liner.

13. A prosthesis as claimed in claim 11, **characterized in that** the liner has been chemically compacted.

14. A prosthesis as claimed in claim 11, **characterized in that** the circumferentially oriented fiber comprises amorphous zones within the oriented polymer crystalline structure, the amorphous zones resulting at least in part from compaction.

15. A prosthesis as claimed in claim 1, **characterized in that** the radially expansible tubular frame defines an axial direction and a circumferential direction; and the plastically expansible liner comprises:
circumferentially oriented yarn (122) comprising at least about 60 Denier fully drawn polyester which has been chemically compacted in a solution comprising between about 92 and 100% methylene chloride and between about 0% and 8% hexafluoro 2-propanol for a time between about 1 and 10 minutes; and
axially oriented yarn (124) comprising less than about 60 Denier polyester, wherein at least an end portion of the liner is woven with less than about 160 ends per inch, wherein the liner expands by at least about 15%, and wherein a porosity of the liner is less than about 1000 ml/min/cm² after expansion.

## Patentansprüche

1. Endoluminare Prothese (10) mit kontrollierter Ausdehnung, die umfasst:
einen radial dehnbaren röhrenförmigen Rahmen (12) mit einer inneren Oberfläche und einer äußeren Oberfläche; und
eine Verkleidung (74), die auf der inneren und/oder der äußeren Oberfläche des Rahmens (12) angeordnet ist;
**dadurch gekennzeichnet, dass**
die Verkleidung (74) plastisch dehnbar ist und der Rahmen (12) und/oder die Verkleidung (74) ein Verstärkungselement (96) aufweisen, das die radiale Ausdehnung der Prothese (10) auf eine vorgegebene ausgedehnte Größe begrenzt.

2. Prothese nach Anspruch 1, **dadurch gekennzeichnet, dass** der Rahmen (12) mehrere Verstärkungselemente (96) aufweist, die die radiale Ausdehnung des Rahmens (12) beschränken, so dass der Rahmen (12) die vorgegebene Größe definiert.

3. Prothese nach Anspruch 2, **dadurch gekennzeichnet, dass** die Verstärkungselemente (96) die elastische Ausdehnung des Rahmens (12) bei der vorgegebenen Größe lösbar beschränken, wobei der Rahmen (12) eine selbstausdehnende Struktur aufweist.

4. Prothese nach Anspruch 2, **dadurch gekennzeichnet, dass** die Verstärkungselemente (96) die plastische Ausdehnung des Rahmens (12) auf die vorgegebene Größe begrenzen, wobei der Rahmen (12) eine plastisch dehnbare Struktur aufweist.

5. Prothese nach Anspruch 1, **dadurch gekennzeichnet, dass** das Verstärkungselement ein Füllelement (122) der Verkleidung umfasst, so dass die Verkleidung die vorgegebene ausgedehnte Größe definiert.

6. Prothese nach Anspruch 5, **dadurch gekennzeichnet, dass** das Füllelement (122) ein Verbundgarn (130) umfasst, das eine nicht dehnbare gewundene Faser (134) und eine zweite Faser (132) enthält, wobei sich die nicht dehnbare Faser während der Ausdehnung der Verkleidung auf die vorgegebene ausgedehnte Größe geradebiegt.

7. Prothese nach Anspruch 1, **dadurch gekennzeichnet, dass** das Verstärkungselement (102) bricht oder fließt, wenn eine radiale Ausdehnungskraft, die eine vorgegebene Schwellenkraft übersteigt, ausgeübt wird, wodurch der Prothese (10) ermöglicht wird, über die vorgegebene ausgedehnte Größe hinaus ausgedehnt zu werden.

8. Prothese nach Anspruch 1, **dadurch gekennzeichnet, dass** die plastisch dehnbare Verkleidung wenigstens über einem Abschnitt der inneren oder der äußeren Oberfläche des Rahmens (12) angeordnet ist und ein in Umfangsrichtung orientiertes Garn umfasst, das eine vollständig gestreckte Faser enthält, die einen maximalen ausgedehnten Umfang der Verkleidung definiert.

9. Prothese nach Anspruch 8, **dadurch gekennzeichnet, dass** die vollständig gestreckte Faser um eine zweite Faser gewickelt ist, wobei der maximale Umfang durch Geradebiegen der vollständig gestreckten Faser definiert ist.

10. Prothese nach Anspruch 1, **dadurch gekennzeichnet, dass** die plastisch dehnbare Verkleidung ein Textilrohr umfasst, das Verbundgarne (130) enthält, die eine nicht dehnbare gewundene Faser (134) und eine dehnbare Faser (132) besitzen, so dass sich die nicht dehnbare Faser (134) während der Ausdehnung des Transplantats geradebiegt, um die Ausdehnung des Textilmaterials über eine vorgegebene Grenze hinaus zu verhindern.

11. Prothese nach Anspruch 1, **dadurch gekennzeichnet, dass** der radial dehnbare röhrenförmige Rahmen eine axiale Richtung und eine Umfangsrichtung definiert; und die plastisch dehnbare Verkleidung ein Textilrohr umfasst, das eine in Umfangsrichtung orientierte Faser (122) enthält, die chemisch verdichtet worden ist.

12. Prothese nach Anspruch 11, **dadurch gekennzeichnet, dass** die in Umfangsrichtung orientierte Faser vor der Bildung der Rohrverkleidung chemisch verdichtet worden ist.

13. Prothese nach Anspruch 11, **dadurch gekennzeichnet, dass** die Verkleidung chemisch verdichtet worden ist.

14. Prothese nach Anspruch 11, **dadurch gekennzeichnet, dass** die in Umfangsrichtung orientierte Faser innerhalb der orientierten kristallinen Polymerstruktur amorphe Zonen umfasst, die sich wenigstens teilweise aus der Verdichtung ergeben.

15. Prothese nach Anspruch 1, **dadurch gekennzeichnet, dass** der radial dehnbare röhrenförmige Rahmen eine axiale Richtung und eine Umfangsrichtung definiert; und die plastisch dehnbare Verkleidung umfasst:
ein in Umfangsrichtung orientiertes Garn (122), das wenigstens ein vollständig gestrecktes Polyester mit etwa 60 Denier enthält, das in einer Lösung, die Methylenchorid in einer Menge im Bereich von etwa 92 bis 100 % und Hexafluor-2-Propanol in einer Menge im Bereich von etwa 0 bis 8 % enthält, während einer Dauer im Bereich von etwa 1 bis 10 Minuten chemisch verdichtet worden ist; und
ein axial orientiertes Garn (124), das einen Polyester von etwa 60 Denier enthält, wobei wenigstens ein Endabschnitt der Verkleidung mit weniger als etwa 160 Enden pro Zoll gewebt ist, wobei sich die Verkleidung um wenigstens etwa 15 % ausdehnt, und wobei eine Porosität der Verkleidung nach der Ausdehnung kleiner als etwa 1000 ml/min/cm² ist.

## Revendications

1. Prothèse endoluminale à extension contrôlée (10) comprenant :
un cadre tubulaire extensible radialement (12) ayant une surface interne et une surface externe ; et
une doublure (74) disposée sur au moins l'une des surfaces interne et externe du cadre (12) ;
**caractérisée en ce que**
la doublure (74) est extensible, sur le plan plastique, et au moins l'un des cadre (12) et doublure (74) comporte un élément de renfort (96) qui limite l'extension radiale de la prothèse (10) jusqu'à une taille prédéterminée en extension.

2. Prothèse selon la revendication 1, **caractérisée en ce que** le cadre (12) comporte une pluralité d'éléments de renfort (96) qui limitent l'extension radiale du cadre (12), de telle sorte que le cadre (12) définit la taille prédéterminée.

3. Prothèse selon la revendication 2, **caractérisée en ce que** les éléments de renfort (96) limitent, de manière dégageable, l'extension élastique du cadre (12) à la taille prédéterminée, le cadre (12) comprenant une structure auto-extensible.

4. Prothèse selon la revendication 2, **caractérisée en ce que** les éléments de renfort (96) limitent l'extension, sur le plan plastique, du cadre (12), à la taille prédéterminée, le cadre (12) comprenant une structure extensible sur le plan plastique.

5. Prothèse selon la revendication 1, **caractérisée en ce que** l'élément de renfort comprend un élément de remplissage (122) de la doublure, de telle sorte que la doublure définit une taille prédéterminée en extension.

6. Prothèse selon la revendication 5, **caractérisée en ce que** l'élément de remplissage (122) comprend un fil composite (130) comportant une fibre inextensible en serpentin (134) et une seconde fibre (132), et dans laquelle la fibre inextensible se redresse au cours de l'extension de la doublure jusqu'à la taille prédéterminée en extension.

7. Prothèse selon la revendication 1, **caractérisée en ce que** l'élément de renfort (102) casse ou cède lorsqu'une force d'extension radiale supérieure à une force seuil prédéterminée est appliquée, ce qui permet que la prothèse (10) connaisse une extension au-delà de la taille prédéterminée en extension.

8. Prothèse selon la revendication 1, **caractérisée en ce que** ladite doublure extensible sur le plan plastique est disposée sur au moins une partie de la surface interne ou externe du cadre (12), qui comprend un fil orienté suivant la circonférence comportant une fibre totalement étirée qui définit un périmètre en extension maximum de la doublure.

9. Prothèse selon la revendication 8, **caractérisée en ce que** la fibre totalement étirée est enroulée autour d'une seconde fibre, et dans laquelle le périmètre maximum est défini en redressant la fibre totalement étirée.

10. Prothèse selon la revendication 1, **caractérisée en ce que** la doublure extensible sur le plan plastique, comprend un tube de tissu comportant des fils composites (130) ayant une fibre inextensible en serpentin (134) et une fibre extensible (132), de telle sorte que la fibre inextensible (134) se redresse au cours de l'extension de la greffe, afin d'empêcher l'extension du tissu au-delà d'une limite prédéterminée.

11. Prothèse selon la revendication 1, **caractérisée en ce que** le cadre tubulaire extensible radialement définit une direction axiale et une direction suivant la circonférence ; et **en ce que** la doublure extensible sur le plan plastique comprend un tube de tissu qui comporte une fibre orientée suivant la circonférence (122) qui a été compactée chimiquement.

12. Prothèse selon la revendication 11, **caractérisée en ce que** la fibre orientée suivant la circonférence a été compactée chimiquement avant de former la doublure en tube.

13. Prothèse selon la revendication 11, **caractérisée en ce que** la doublure a été compactée chimiquement.

14. Prothèse selon la revendication 11, **caractérisée en ce que** la fibre orientée suivant la circonférence comprend des zones amorphes à l'intérieur de la structure cristalline du polymère, les zones amorphes résultant, au moins en partie, du compactage.

15. Prothèse selon la revendication 1, **caractérisée en ce que** le cadre tubulaire extensible radialement définit une direction axiale et une direction de circonférence ; et **en ce que** la doublure extensible sur la plan plastique comprend :
un fil orienté suivant la circonférence (122) comprenant un polyester totalement étiré d'au moins 60 Deniers, qui a été compacté chimiquement dans une solution comprenant entre environ 92 % et 100 % de chlorure de méthylène et entre environ 0 % et 8 % d'hexafluoro 2-propanol pendant un laps de temps compris entre environ 1 minute et 10 minutes ; et
un fil orienté axialement (124) comprenant un polyester de moins d'environ 60 Deniers,
dans laquelle au moins une partie d'extrémité de la doublure est tissée avec moins d'environ 160 fils par pouce, dans laquelle la doublure connaît une extension d'au moins environ 15 % et dans laquelle une porosité de la doublure est inférieure à environ 1000 ml/mn/cm², après l'extension.
